Europäisches Patentamt

(19) European Patent Office    (11) Publication number: **0 047 185**

Office européen des brevets    **B1**

(12) **EUROPEAN PATENT SPECIFICATION**

(45) Date of publication of patent specification: **11.12.85**    (51) Int. Cl.⁴: **A 61 M 16/01**

(21) Application number: **81304018.5**

(22) Date of filing: **02.09.81**

(54) **Anaesthetic system.**

(30) Priority: **02.09.80 ZA 805418**

(43) Date of publication of application:
**10.03.82 Bulletin 82/10**

(45) Publication of the grant of the patent:
**11.12.85 Bulletin 85/50**

(84) Designated Contracting States:
**AT BE CH DE FR GB IT LI LU NL SE**

(56) References cited:
**FR-A-2 385 408**
**GB-A- 734 639**
**GB-A-2 029 703**
**US-A-3 814 092**
**US-A-3 993 059**
**US-A-4 007 736**
**US-A-4 249 527**

**The file contains technical information
submitted after the application was filed and
not included in this specification**

(73) Proprietor: **Humphrey, David
222 Chelmsford Road
Durban 4001 Natal (ZA)**

(72) Inventor: **Humphrey, David
222 Chelmsford Road
Durban 4001 Natal (ZA)**

(74) Representative: **Baillie, Iain Cameron et al
c/o Ladas & Parry Isartorplatz 5
D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to anaesthetic apparatus and in particular to anaesthetic breathing systems.

The term "anaesthetic system" or anaesthetic breathing system, when used in this specification, is taken to mean the connecting apparatus between the anaesthetic machine and the face mask or endotracheal tube which connects to a patient, except where the context indicates otherwise.

At present three systems which do not use recycling of anaesthetic gases through soda lime are widely in use, each finding application in one or two of the four circumstances in which inhalational agents are used. The Mapleson A or Magill system, is used for spontaneous respiration on adults and comprises a flexible tube delivering fresh gas from the machine, a reservoir bag at the machine end, and a pop-off valve, which is a simple relief valve, adjacent the patient's face. For controlled ventilation on adults, the Mapleson D or Bain system is used. This system comprises a fresh gas supply tube located within the bore of an expired gas removal tube. The outer tube is connected to the ventilator from where the expired gases may be removed from the theatre.

In paediatrics, a simple T-piece (Mapleson E) is generally used for both spontaneous respiration and controlled ventilation.

It is clear that each particular system is of limited application. In addition, the Magill and the T-piece systems contribute to the atmospheric pollution of the theatre by the introduction of expired respiration products and anaesthetic gas into the theatre, only the Bain system allowing easy scavenging.

A later system, based on the Mapleson A principle and developed by Lack, addressed the problem of theatre pollution by the provision of a take-off tube for expired gases co-axially down the fresh gas supply tube. The take-off tube exits from the bore of the supply tube at a location remote from the patient where a relief valve is arranged to blow off when the expiration pressure of the patient overcomes the resistance of the valve mechanism. The Lack system, can only be used in the same situations as the Magill.

Applicant is aware of the following United States Patents: 3,993,059 (S jostrand) which relates to a high frequency positive ventilation system; 3,814,092 (Simionescu et al) which relates to a portable anaesthetic machine; 4,007,736 (Schreiber) which describes a ventilator with a timing mechanism and 4,249,527 (Ko et al) which relates to a pressure administrating apparatus which maintains a positive pressure in the lungs of a patient and United Kingdom Patents: 734,639) (Svenska Aktiebolaget Gas-accumulator) which relates to a closed and open circuit anaesthetic system and 1,272,583 (Blease Medical Equipment) which describes a ventilating machine designed to facilitate sterilization after use.

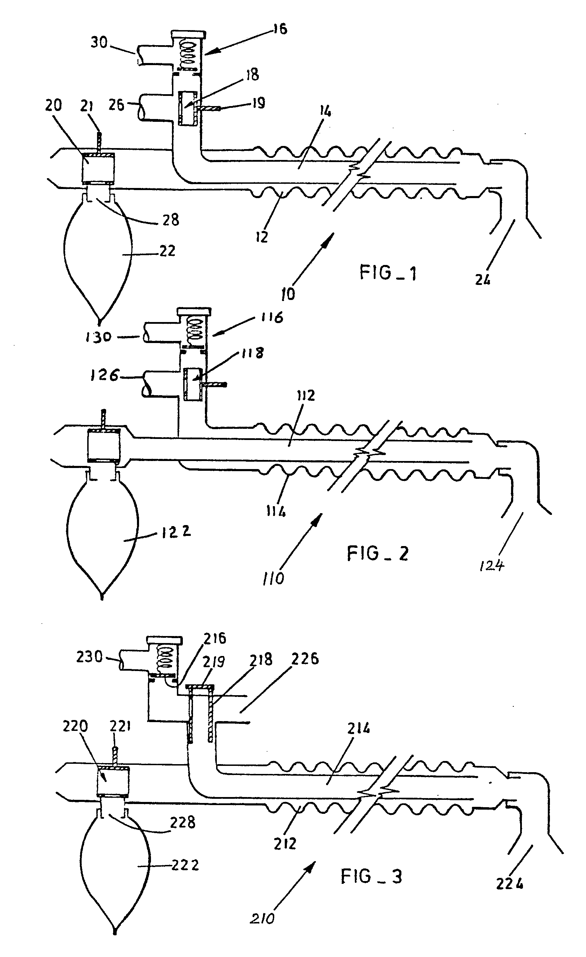

British Patent 2 029 703 (Miller) discloses an anaesthetic system including the features specified in the prior art portion of claim 1. This patent is concerned with a fluid control device (12) at the patient end of the apparatus. This device, to which is attached an anaesthetic gas supply tube and from which an exhaust gas tube passes to the atmosphere, causes anaesthetic gas that lies in the patient's airways to be directed by means of a jet (20) back along the supply tube during expiration. The control device of Miller actually hinders gas flow and thereby worsens the loss of dead space gas. The present invention also seeks to overcome this disadvantage by providing an unhindered passage of gas between the inspiratory and expiratory tubes and the patient. The Sjostrand patent referred to above has no reservoir bag on the inspirator tubes. Sjostrand also requires a pressure valve at the conference of the inspiratory and respiratory tubes (Figure 1, references 9, 11) and also requires a ventilator connected to the inspiratory tube.

It is an object of this invention to provide a system which will find application in each and all of the circumstances mentioned above and which will, moreover, be as efficient, or considerably more efficient, than the systems presently in use in some or all of the applications referred to.

It is a further object of this invention to facilitate the scavenging of waste expired anaesthetic gases in all the applications mentioned above, thus reducing theatre pollution and its consequent danger to theatre staff to minimal levels or even eliminating pollution completely.

Yet another object of this invention is to provide an anaesthetic breathing system suitable for anaesthesia administration in both the sophisticated academic environment and remote rural areas. By virtue of the properties of the apparatus the anaesthetist can use very economical anaesthetic gas flows even without carbon dioxide absorption and yet he does not prejudice the safety of his patient. He can predict that carbon dioxide tension of his patient during controlled ventilation even when the latter is performed by hand. The invention is robust, simple and easily sterilized and consequently has many advantages over the circle absorber system which, unless specially adapted, is also unsuitable for children.

Yet another object of this invention is to provide an apparatus which is cheaper, easier to use, and less bulky than a combination of currently used anaesthetic breathing systems required for anaesthesia in the four circumstances mentioned earlier. Further it eliminates the use of soda lime and thus also the cost, transportation and the difficulties often encountered in remote areas in obtaining it.

According to the invention there is provided an anaesthetic system including a fresh gas supply tube having a port for connection of a reservoir bag being controlled by a switchable valve and an expiratory tube having an outlet controlled by a relief valve, characterised by a second outlet located in the expiratory tube and being controlled by a switchable valve. Special embodiments of the invention are defined in claims 2 to 5 below.

The arrangement of the invention permits a wide variety of applications for adult and child, spontaneous and controlled ventilation circumstances.

The expiratory tube is provided with two outlets; in one form of the invention the first outlet is controlled by a relief valve or a pop-off valve, and the second outlet is controlled by a plug valve.

The port connecting the fresh gas supply tube to the reservoir bag may be controlled by a plug valve.

The fresh gas supply tube and expiratory tube may communicate freely with each other at the patient end and with the patient connector.

The fresh gas supply tube and the expiratory tube may be coaxial and the outlets are preferably located at a position remote from the patient end of the system.

Embodiments of the invention are shown in the accompanying drawings which are partly diagrammatical representations of an anaesthetic system according to the invention.

The anaesthetic system 10 shown in Figure 1 comprises a fresh gas supply tube 12 or inspiratory tube, the patient end of which consists of flexible tubing, and an expired gas removal tube 14, or expiratory tube, which runs down the bore of the fresh gas supply tube 12 and exits therefrom at a location remote from the patient. The expired gas removal tube 14 terminates at the upper end thereof in a main outlet 30 controlled by a relief valve 16 which is a simple pop-off valve. A secondary outlet 26 upstream of the main outlet 30 has a manually operable valve 18; a second manually operable valve 20 is located in the fresh gas supply tube 12 to open or close a port 28 to a reservoir bag 22. Both valves 18, 20 are plug valves located within the tubes, with a central bore disposed co-axially with the principal axis of the tube, each arranged to close their outlets or port in one position of the levers 19, 21 and in the other position of the levers 19, 21 to open the outlet 26 or port 28. The pop-off valve 16 is a simple spring loaded valve with a screw cap which can be screwed in to close the valve 16.

A face mask 24 is shown attached to the patient end of the fresh gas supply tube 12. The expired gas removal tube 14 stops short of the end of the fresh gas supply tube and is supported therein by a set of radially extending vanes (not shown).

In use the settings of the valves 16, 18, 20 will vary depending on the application of the system 10.

For adult spontaneous respiration the system 10 is used in a Mapleson A configuration. The reservoir bag port valve 20 is opened thereby including the reservoir bag 22 in the circuit and the outlet valve 18 is closed so that the relief valve 16 pops off during each expiration cycle of the patient. In this configuration the system can be regarded as a Lack system, fresh gas filling the reservoir bag 22 on the inspiratory limb and passing down the outer tube 12. Expired gases are vented by the inner tube 14 and exhausted through the pop-off valve 16 to be scavenged at the outlet 30.

The efficiency of the system 10 in this configuration has been determined on 20 anaesthetised patients using a fresh gas flow of 70 ml. $kg^{-1}$. $min^{-1}$.

During this test, the applicant found that the system 10, even with a fresh gas flow of only 50 ml. $kg^{-1}$. $min^{-1}$ performed as efficiently as the Magill (Mapleson A system) with a fresh gas flow of 70 ml. $kg^{-1}$. $min^{-1}$.

To change the system 10 for controlled ventilation in both children and adults, the reservoir bag 22 is excluded from the circuit by means of the valve 20. A ventilator is connected to the outlet 26 while the outlet valve 18 is opened and the relief valve 16 is stopped down by screwing the cap down to the closed position. Fresh gas flows directly to the patient via the supply tube 12 and expired gas exits freely through the open outlet valve 18 to the ventilator.

The system functions as a modified Mapleson D system. Like the Bain (which is also modified Mapleson D system) normocarbia can be maintained with hyperventilation and a fresh gas flow of 70 ml. $kg^{-1}$. $min^{-1}$.

When used for either spontaneous or controlled ventilation in healthy adults, the system 10 maintains normocarbia with a fresh gas flow of 70 ml. $kg^{-1}$. $min^{-1}$ or less.

By including or excluding the reservoir bag 22, the system 10 can be quickly converted from a Mapleson D system to an A or vice versa.

For spontaneous respiration in paediatric anaesthesia, the system 10 may be used as a valveless Mapleson A, a system that, in the experience of the applicant, is two to three times more efficient that the traditional T-piece of Mapleson E system.

Fresh gas fills a smaller inspiratory reservoir bag 22 of 1 litre, in place of the adult 2 litre bag. Expired gases pass along the inner tube 14, but instead of being exhausted, as with adults through the pop-off valve 16 which is fully closed, the outlet valve 18 is opened by the lever 19 to allow gas to escape freely without passing through the pop-off valve.

The system 10 required 3,1 times as little fresh gas as the T-piece to prevent rebreathing, an observation which was noted consistently during trials done by the applicant on children breathing spontaneously. It has been found that system 10 requires a maximum fresh gas flow of about 3.5 litres $min^{-1}$ for spontaneous respiration in children.

For controlled ventilation in children the system 10 is used as a Bain (modified Mapleson D) system as described above. A fresh gas flow of 3

litres min$^{-1}$ has been shown to maintain normocarbia or mild hypocarbia.

The resistance to expiration of the system 10 has been found to be remarkably low. It has a resistance of less than half that of 40 Magill systems tested, while for paediatric use it also offers some 50% less resistance than the T-piece.

In Figure 2 the system 10 of the Figure 1 is shown in an "inside out" configuration (110) in which the components function exactly as they do in the system 10 with the exception that the fresh gas now flows down the inner tube 112 while the outer tube 114 serves as the expiratory tube.

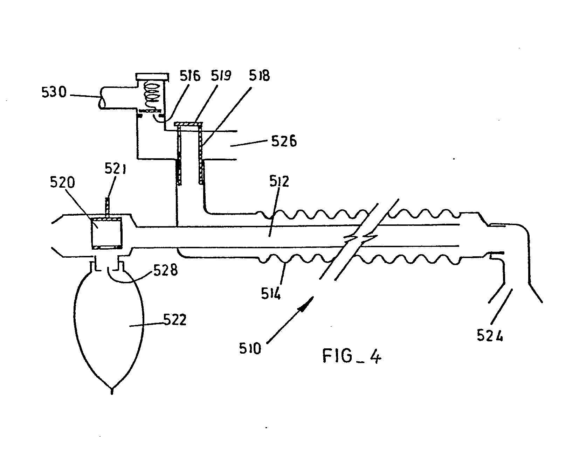

In Figure 3 an alternative valve 218 is provided which has an exterior control 219, the valve allowing expiratory gases to flow either to the valve 216 or through outlet 226 to atmosphere or a ventilator. Except where otherwise indicated the last two digits of each of the reference numerals used in Figures 3 to 6 correspond with the reference numerals used in Figure 1, indicating like components.

Figure 4 is likewise in reversal of Figure 3, the expiratory tube 514 being the outer of the co-axial tubes.

It will be appreciated that the inspiratory and expiratory tubes may lie in configurations other than those illustrated in the drawings. Furthermore soda lime canisters may be incorporated with the two one-way valves and flexible tubing inserted between the expiratory outlet 26 in Figure 1 (or 226 or 526) and port 28 (or 228 or 528). The flow of gases through the one-way valves will be towards port 28 (or 228 or 528). A T-piece connector may be used to connect the reservoir bag 22 (or 222 or 522) and the canister, valves and tubing with port 28 (or 228 or 528).

### Claims

1. Anaesthetic system (10, 110, 210, 510) including a fresh gas supply tube (12, 112, 212, 512) having a port (28, 228, 528), for connection of a reservoir bag (22, 122, 222, 522) being controlled by a switchable valve (20, 220, 520), and an expiratory tube (14, 114, 214, 514) having an outlet (30, 130, 230, 530) controlled by a relief valve (16, 116, 216, 516), characterised by a second outlet (26, 126, 226, 526) located in the expiratory tube (14, 114, 214, 514) and being controlled by a switchable valve (18, 118, 218, 518).

2. Anaesthetic system according to claim 1, characterised in that the fresh gas supply tube (12, 112, 212, 512) and expiratory tube (14, 114, 214, 514) communicate freely with each other at the patient end and with the patient connector (24, 124, 224, 524).

3. Anaesthetic system according to claim 1 or 2, characterised in that the valve of the second outlet (26) is a plug valve (18).

4. Anaesthetic system according to claim 3, characterised in that the relief valve (16, 116, 216, 516) is a pop-off valve.

5. Anaesthetic system according to any of claims 1 to 4, characterised in that the reservoir bag (22, 122, 222, 522) is connected to the fresh gas supply tube (12, 112, 212, 512) through a plug valve (20, 220, 520).

### Revendications

1. Système pour anesthésie (10, 110, 210, 510) comprenant un tube d'alimentation de gaz frais (12, 112, 212, 512) comportant un orifice (28, 228, 528) à raccorder à un ballon formant réservoir (22, 122, 222, 522) qui est commandé par une valve commutable (20, 220, 520) et une tube d'expiration (14, 114, 214, 514) comportant une sortie (30, 130, 230, 530) commandée par une valve de décompression (16, 116, 216, 516), caractérisé par une seconde sortie (26, 126, 226, 526) installée dans le tube d'expiration (14, 114, 214, 514) et commandée par une valve commutable (18, 118, 218, 518).

2. Système pour anesthésia suivant la revendication 1, caractérisé en ce que le tube d'alimentation de gaz frais (12, 112, 212, 512) et le tube d'expiration (14, 114, 214, 514) communiquent librement l'un avec l'autre à l'extrémité située du côté du patient et avec le raccord au patient (24, 124, 224, 524).

3. Système pour anesthésie suivant la revendication 1 ou 2, caractérisé en ce que la valve de la seconde sortie (26) est une valve à tournant (18).

4. Système pour anesthésie suivant la revendication 3, caractérisé en ce que la valve de décompression (16, 116, 216, 516) est une valve de sécurité.

5. Système pour anesthésie suivant l'une quelconque des revendications 1 à 4, caractérisé en ce que le ballon formant réservoir (22, 122, 222, 522) est raccordé au tube d'alimentation de gaz frais (12, 112, 212, 512) par l'intermédiaire d'une valve à tournant (20, 220, 520).

### Patentansprüche

1. Anästhesiesystem (10, 110, 210, 510) mit einer Frischgaszuleitung (12, 112, 212, 512), die einen Anschluß (28, 228, 528) zum Verbinden mit einem Druckgasbeutel (22, 122, 222, 520) unter Steuerung durch ein schaltbares Ventil (20, 220, 520) besitzt, und mit einer Ausatemleitung (14, 114, 214, 514), die einen Auslaß (30, 130, 230, 530) besitzt, der durch ein Druckbegrenzungsventil (16, 116, 216, 516) gesteuert wird, gekennzeichnet durch einen zweiten Auslaß (26, 126, 226, 526), der in der Ausatemleitung (14, 114, 214, 514) angeordnet ist und von einem schaltbaren Ventil (18, 118, 218, 518) gesteuert wird.

2. Anästhesiesystem nach Anspruch 1, dadurch gekennzeichnet, daß die Frischgaszuleitung (12, 112, 212, 512) und die Ausatemleitung (14, 114, 214, 514) an dem patientenseitigen Ende miteinander und mit dem zum Patienten führen den Verbinder (24, 124, 224, 524) frei in Verbindung stehen.

3. Anästhesiesystem nach Anspruch 1 oder 2, dadurch gekennzeichnet, daß das dem zweiten Auslaß (26) zugeordnete Ventil ein Kegelventil (18) ist.

4. Anästhesiesystem nach Anspruch 2, dadurch gekennzeichnet, daß das Druckbegrenzungsventil (16, 116, 216, 516) ein Federsicherheitsventil ist.

5. Anästhesiesystem nach einem der Ansprüche 1 bis 4, dadurch gekennzeichnet, daß der Druckgasbeutel (22, 122, 222, 522) mit der Frischgaszuleitung (12, 112, 212, 512) durch ein Kegelventil (20, 220, 520) verbunden ist.

FIG _ 1

FIG _ 2

FIG _ 3

FIG_4